# EUROPEAN PATENT SPECIFICATION

(11) **EP 0 961 780 B1**
(45) Date of publication and mention of the grant of the patent: **11.04.2007**
(21) Application number: 98905576.9
(22) Date of filing: 12.02.1998
(51) Int. Cl.: G01N 33/574

(54) **PROTEIN MARKERS FOR LUNG CANCER AND USE THEREOF**
PROTEINMARKER FÜR LUNGENKREBS UND DEREN VERWENDUNG
PROTEINES-MARQUEURS POUR LE CANCER DU POUMON ET UTILISATION DE CES DERNIERES

(30) Priority: 12.02.1997 US 38819 P
(43) Date of publication of application: 08.12.1999
(73) Proprietor: Electrophoretics Limited, Cobham Surrey KT11 3EP (GB); THE REGENTS OF THE UNIVERSITY OF MICHIGAN, Ann Arbor, Michigan 48109-1280 (US)
(72) Inventor: HANASH, Samir, M. University of Michigan, Ann Arbor, MI 48109-0752 (US)
(74) Representative: Lucas, Brian Ronald
(86) International application number: PCT/IB1998/000361
(87) International publication number: WO 1998/035985

(56) References cited:
- EP-A- 0 184 906
- EP-A- 0 585 201
- EP-A- 0 695 760
- US-A- 5 019 497
- US-A- 5 171 665
- T. HIRANO ET AL: "Detection of polypeptides associated with the histopathological differentiation of primary lung carcinoma" BRITISH J. CANCER, vol. 72, 1995, pages 840-848, XP002070782
- B. FRANZEN ET AL: "Two-dimensional polyacrylamide gel-electrophoresis of human lung cancer" ELECTROPHORESIS, vol. 12, 1991, pages 509-515, XP002070783
- Database: Emest9 ID: HSAA83401 AC:AA181619 Homo sapiens cDNA clone 613065 5' XP002070784
- Database: Emest8 ID: HS845338 AC:W24845 Homo sapiens cDNA clone 308303 5' XP002070785

## Description

### Background of the Invention

### Field of the Invention

The present invention relates to an in-vitro method for diagnosing lung cancer.

A large number of polypeptides that are differentially expressed between the three major lung tumor types have been identified. A small number of these polypeptides overlap with markers previously identified as markers for esophageal tumors However, the majority (some thirty polypeptides) are new to the present analysis.

### Description of Related Art

Lung cancer is the major cause of cancer deaths in men over 35 years of age and is a leading cause of death In women in this age group There are several sub-types of lung cancer. Squamous cell carcinoma, adenocarcinoma and small cell carcinoma represent major sub-types. In view of the overall high incidence and mortality of lung cancer, approaches to screen and detect this type of cancer at an early stage would be quite beneficial. However the benefits of currently available screening strategies are doubtful and there remains much need for more effective strategies. To that effect, the identification of biochemical markers With a high degree of specificity for tumors and specific subtypes of tumors would be beneficial.

At the present time, lung cancer is diagnosed primarily by biopsy. Unfortunately, by the time the cancer is diagnosed it is often far advanced. Survival after diagnosis is poor.

Thus, a need exists for the diagnosis of lung cancer at an early stage. Markers which correspond to the advance of the illness may be used to monitor therapeutic regimens.

T. Hirano et al, British J. Cancer, vol. 72, 1995, pages 840-848, relates to detection of polypeptides associated with the histopathological differentiation of primary lung carcinoma.

B. Franzen et al, Electrophoresis, vol. 12, 1991, pages 509-515. relates to two-dimensional polyacrylamide gel electrophoresis of human lung cancer.

EP-A-0 585 201 relates to the measurement of MRP8, MRP14 and the heterodimer of MRP 8/14 in the serum of patients with inflammatory disease.

### Summary of the Invention

The present invention provides a method for diagnosing lung cancer in an animal or human, comprising detecting at least one of the proteins MRP8 and MRP14 which is overexpressed in lung tumours, in a serum sample or a tissue section from the animal or human, but excluding a diagnostic method practised on the human or animal body.

The strategy of the present invention involves analyzing several hundred cellular proteins expressed in different lung cancer sub-types to identify proteins that are subtypes(s) specific. Using the procedure of two-dimensional gel electrophoresis, a subset of proteins that appear to distinguish between the major sub-types in a statistically significant manner has been detected. These proteins have utilities in many areas, including the following:
1. Screening normal individuals or individuals at an increased risk for lung cancer.
2. Establishing the specific lung cancer sub-type at the time of diagnosis.
3. Providing an indication of prognosis for individuals diagnosed with a specific lung cancer sub-type.
4. Providing novel approaches for therapy, based on understanding of the role of these proteins in different lung cancer sub-types.

By comparison of 2-D gels showing proteins from normal lung and different types of lung tumors such as squamous, small cell, and adenocarcinoma, a set of proteins have been identified in the different source tissues. These proteins provide information on the pathogenesis of lung tumors, and have utility as markers to monitor therapeutic regimens. The proteins can also be purified and used as immunogens to generate antibodies which can be used as diagnostic reagents. In addition, some of the proteins or antibodies thereto may have therapeutic applications.

### Brief Description of the Drawings

Figure 1 shows an lsoelectric-Focusing (IEF) gel of a sample from a patient with Squamous cell lung cancer.
Figure 2 shows an IEF gel of a sample from a patient with classical small cell lung cancer.
Figure 3 shows an IEF gel of a sample from a patient with adenocarcinoma of the lung.

### Detailed Description of the Invention

One aspect of the invention is a new diagnostic method for lung tumors. The diagnostic method is based on the detection of at least one protein which is overexpressed in lung tumors relative to non-tumor lung tissues and which is specific for a lung tumor sub-type. In order to identify the protein(s) to be used in lung tumor diagnosis, proteins expressed in 60 lung tumors were analyzed using 2-D gel electrophoresis. By comparing the , protein gel electrophoresis profiles of lung tumors and non-tumor lung tissues, proteins which are overexpressed in lung tumors were located. As demonstrated below, some of the specific proteins over-expressed also correlate with the lung tumor sub-type. Therefore, by concentrating on a plurality of protein markers which are overexpressed in different specific lung tumor subtypes, a diagnosis of the lung tumor sub-type can be made. For instance, relying on at least three protein markers each specific for one of three major lung tumor subtypes, i.e. squamous cell carcinoma, adenocarcinoma or small cell carcinoma, a diagnosis of the major lung tumor subtype can be made. It should be emphasized that the protein markers can be determined using gel electrophoresis in the absence of antibodies, an immunoassay if antibodies specific for the protein markers are available or any other method of detecting the protein markers. Antibodies specific for the protein markers allow *in vitro* or *in vivo* applications of the diagnostic method.

Another aspect of the invention is a method to monitor the progress of treatment of lung tumors by monitoring the appearance of at least one specific protein marker for the lung tumor sub-type being treated. Some of the protein markers identified in the instant invention can be monitored during the course of treatment of a lung tumor with an emphasis on the protein markers specific for the lung tumor sub-type under treatment. As the treatment progresses, the presence of at least one of these specific protein markers can be followed as another way to judge the treatment effectiveness.

### Carrier ampholyte (CA) based 2-D gels of lung cancer.

Tissue from over 60 lung tumors was obtained for 2-D analysis.

Most of the tumors have a pair of replicate silver stained gels available, in which the first dimension gel was an iso-electric focusing gel. In addition, most of the tumors were analyzed using immobilized pH gradients. The common tumor types are well represented: classical Small Cell (SC), Adenocarcinoma (Ad) and Squamous (Sq) tumors of the lung. Rarer tumor types were represented by fewer samples.

The analysis of the three main lung tumor types employed visual analysis of 3 large batches of gels that contained the largest numbers of the tumor types of interest (more than 10 of each of the three types). Images were also studied on the computer, one small close-up section at a time, matching those spots between images that appears to hold the most promise on a subset of the very best images. For the computerized analysis, spots were matched to image Ab6148, a SC sample, from which the "lung" spot numbering system used here is derived. This master is also matched to the master image used in the tumor studies including esophagus, colon, pancreas, leukemia, brain and breast tumors, so that each spot of interest in lung also has a spot number in the other systems. At the time spots of interest were identified, comments about each spot were made, largely concerning which samples had the largest or smallest spots.

It appears that certain sets of interesting spots should be treated as groups, that is, that they are likely to be the product of a single gene, differing only in their post-translational modification. This interpretation is based on the proximity of the spots on the gel, the geometry of the constellation that they form (e.g., a "charge chain"), their identical color with silver staining, and the fact that the quantities in different samples are correlated. In such cases, only a single spot has been selected for quantitation, typically the largest in the group or the spot that exhibits the least overlap with other spots thought to be unrelated. The groups and the representatives chosen for quantitation are:

| **Group** | **Spot Quantitated** |
|---|---|
| 37-40 | 40 |
| 28-30 | 29 |
| 52-54 | 53 |
| 33-35 | 33 |
| 87-89 | 87,88 the P18 protein spots |

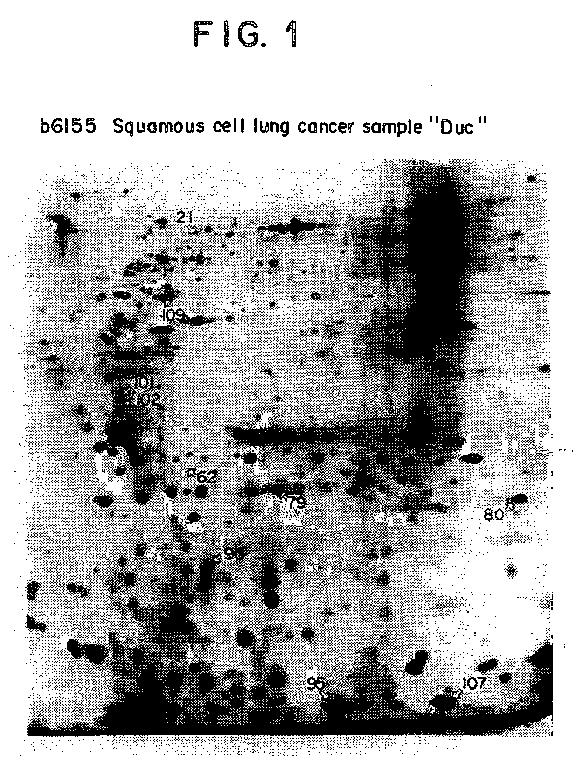

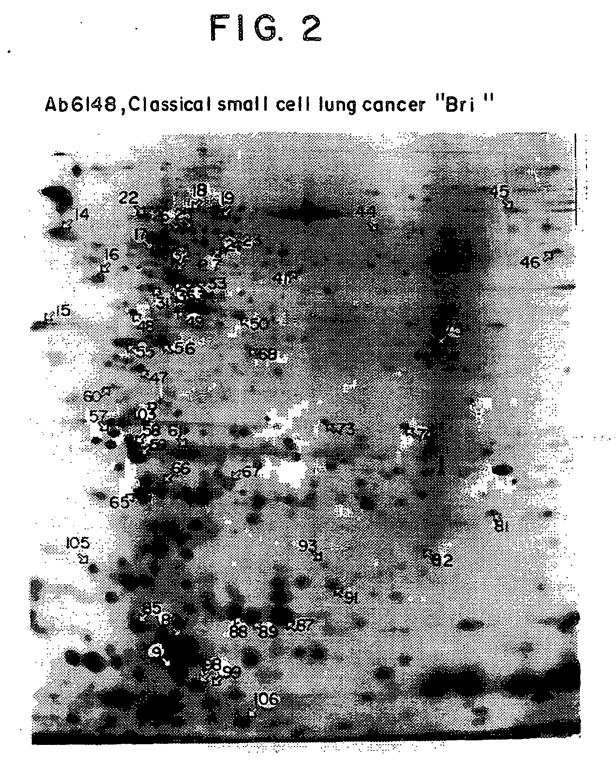

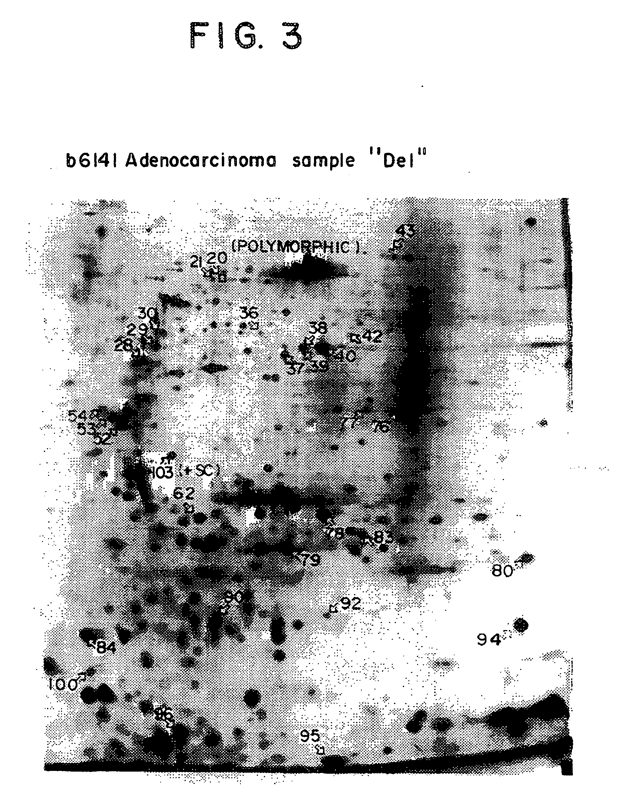

Figures 1-3 show the location of the candidate spots. These are labeled with spot numbers specific to the lung tumor matching.

Carrier ampholyte-based 2-D gels that cover the pH range of approximately 3.5-10.0 were prepared for all specimens.

Tissue was solubilized by addition of lysis buffer consisting of (per liter) 8 M urea, 20 ml of Nonidet P-40 surfactant, 20 ml of ampholytes (pH 3.5-10), 20 ml of 2-mercaptoethanol, and 0.2 mM of phenylmethylsulfonyl fluoride in distilled deionized water. Approximately 30 µl aliquots containing 70 µg of protein were loaded on individual gels.

Because isoelectric focusing is sensitive to charge modification, it is important to minimize protein alterations (e.g., proteolysis, deamidation of glutamine and asparagine, oxidation of cystine to cystic acid, carbamylation) that can result from improper sample preparation. Once solubilized, samples may be stored frozen at -80°C for short periods (<1 month) without significant protein modification).

2-D PAGE was done as previously described (Strahler et al, *Journal of Clinical Investigation,* 85:200-207, 1990). In most cases aliquots were immediately applied onto isofocusing gels. First-dimension gels contained 50 ml of ampholytes per liter (pH 3.5-10). Isofocusing was done at 1,200 V for 16 h and 1,500 V for the last 2 h. 20 gels were run simultaneously. For the second-dimension separation, an acrylamide gradient of 11.4-14.0 g/dl was used. Protein spots in gels were visualized by the silver-staining technique of Merril et al. (Merril et al, Science, 211:1437-1438, 1981).

### Immobilized pH gradient (IPG) 2-D gels of lung cancer

In addition to generating 2-D patterns that were carrier ampholyte-based, a second set of patterns using immobilized pH gradients were generated for many of the tumors.

Samples were prepared as for the CA based 2-D gels of lung cancer discussed above. For first dimension separation an immobilized pH gradient covering the separation range of pH 4-10. The second dimension is the same as for the CA based 2-D gels.

IPG gels are prepared using derivatives of acrylamide having carboxyl or tertiary amino groups with specific pK values. A linear pH gradient is prepared from a dense, acidic solution and a light, basic solution using a two-chamber microgradient former. The pH gradient is stabilized during polymerization of the Immobiline-acryl-amide-bisacrylamide matrix by a colinear gradient of glycerol. Formulations of buffering Immobiline mixtures with titrating Immobiline for the pH limit solutions for narrow pH gradients (1 pH unit) or for broad pH gradients (>1 pH unit, up to 6 pH units) (Gianazza et al, Electrophoresis 6:113 (1985) and LKB application Note 324 (1984)) have been published.

The second dimension separates proteins on the basis of molecular weight in an SDS gel. An 11.5 to 14% T (2.6% cross-linking) acrylamide gradient provides effective separation of proteins of mass from 15,000 to 100,000., Proteins outside this range are less well resolved. Proteins with molecular weight less than 10,000 Da electrophorese close to the dye front and are not resolved.

### Computer assisted analysis of 2-D gels

Each gel was scanned in a 1024 X 1024 pixel format, where each pixel can have one of 256 possible values representing different degrees of intensity. Spot lists for study images are matched to spot lists of master images so that the result is a hierarchy of matched protein spots. The purpose of the matching is to link the same polypeptide spot through the hierarchy to allow assessment of its presence, quantitative variation and specificity, as described in Strahler et al., 1990. For comparison of the amount of individual proteins between gels, an adjustment process is utilized. The integrated intensity of detected polypeptides, measured in units of optical density per square millimeter, is adjusted relative to the intensity of reference polypeptides that are ubiquitously expressed. The adjustment is made to compensate for any variation between gels due to protein loading or staining.

Most spots of interest were quantitated and the results are shown in Tables 1-5. A few spots that appear in Figures 1-3 as interesting do not appear in the Tables. Factors for not including spots are:
- They are part of a larger family of spots as explained above.
- Interest in them diminished after the quantitation results were analyzed (e.g., lung 32, 44, 46, 99).
- They have been studied previously. This includes lung spot numbers 23-26 (np65's), 56 (B23's), 87-89 (P18's), 97 (CRBP-1), 60 (PCNA), 78 (Hsp27), as well as NDPK-A. A few of these famous spots were quantitated to help characterize each tumor sample (P18, P18a, CRBP-I, Hsp27, Hsp27a).

### Assessment of spots in other tissues

A variety of normal tissues and tumors have been studied in an effort to gain some insight into the spots found interesting in lung tumors. The spots included in the list below represent that subset of spots that were quantitated and are considered very interesting. Some quantitated spots are considered less interesting at this time because the differences between lung tumors were not statistically very significant, the mean differences between tumor types were not very large, or because the spots did not appear very much larger in tumors than in control lung samples.

Some spots are still included even though they did not give very small P-values. Usually this is because it is believed that there is potentially an interesting difference, but the fairly simple statistical tests employed are ignoring group (gel batch) effects or are affected by a few cases where the samples do not all agree perfectly (inflated variance measures). It was also in a spot's favor if it had been identified as interesting in previous studies, including studies of esophagus tumors.
GELS:
Brain: Medulloblastoma, Glioblastoma, and normal samples.
Breast Tumors.
Leukemias: AML=ANLL, CALL and normal PBL's
Lung Tumors: Squamous (Squ), Small Cell (SC), Adenocarcinomas (Adn) and normal lung samples (NM).
Neuroblastomas:Various stages and myc copy numbers.
Esophagus: Squamous Carcinomas of the Esophagus (SC), normal esophagus (NE), gastric mucosa (GM), Barrett's (BA), esophageal adenocarcinoma (EA) and tumor of the cardia (TC).
Entries:
   - L =: Large, as big or bigger than in Esophageal adenocarcinoma or Tumor of the Cardia.
   - M =: Medium, there but not as big as in tumors of interest.
   - S =: Small
   - A =: Absent

S? or A? indicates inability to identify the spot in some tissue, simply because there is nothing like what was seen in the tumors in the area. Conversely L? means there is a big spot in the location, but it is uncertain whether it is the sample spot. A * indicates that there is a note below.

The first spot numbers are those used in matching lung tumors (Ab6148). The second spot numbers are from the master image from esophagus (Bb9779). A "@" by esophagus indicates that the spot was noted as interesting in that esophageal tissue. There are sometimes notes for these spots in esophagus samples in other reports. One general observation is that it is easiest to compare SC lung with neuroblastomas.

The first block of spots was initially thought to be larger in SQ or Ad lung (usually Ad) while the second block of spots was thought larger in SC lung samples. The quantitative results should be used to judge the exact status with regard to spot sizes in the different sample types, since sometimes a spot is larger in two of the types, or has a pattern of being largest in one type, smallest in another, and intermediate in the third tumor type.

### Spot quantitation for lung tumors.

Spots in digital images of Lung Squamous tumors (Sq), Adenocarcinoma tumors (Ad), and Small Cell Lung cancers (SC) from 3 runs of IEF gels were quantitated. There were 9 Sq, 8 Ad and 9 SC samples in total. Sources of the samples were primary tumors (PT) or metastatic (MT). The groups of gels formed by electrophoretic runs are labeled, A, B and C in the first column of the table. "Stage" of the tumor is labeled under "stg".

The gels with images matched to a master lung pattern were largely those from the group labeled "A". Some spots were omitted because they are difficult to quantitate, because they seem to be a member of a family of spots only one of which appears in the table below, or because they are already known. Ten reference spots that appear to be more or less invariable between sample types were also quantitated, for use in adjusting the spot integrated intensity data. The spots are labeled in the order of another table in which other tissue types were surveyed. Four "famous spots" (L2 = phosphorylated Hsp27, L4 = unphosphorylated Hsp27. P18 and P18a = phosphorylated P18) are also included to help characterize the samples.

Gel to gel adjustment using the ten reference spots was by what has become the usual method. A standard was formed by computing the average size of each spot across the gels in this study. To compute the adjustment for a particular gel, the ratios of each spot on the gel to the standard were calculated and the ratios were averaged (by taking antilogarithms of the average log ratio). Raw spot integrated intensities are divided by this adjustment factor to obtain the adjusted integrated intensities tabled below. For each gel the adjustment factor is tabled under "Dark".

For each spot the means and variances with each sample type are given as well as the p-value for an F-test of whether the 3 means are identical. There appear to be run ejects and individual effects for some spots, which should probably be judged by eye, and this run effect is why the data is tabled in blocks according to groups formed by electrophoretic runs. Often one can see that the significance for tests considering group effects would be greater, or that omitting a single individual with an enormous value would reduce the variances enough to change the P-value considerably.

### Antibody production

The proteins eluted from the gels, or peptide fragments thereof, may be used as immunogen for the production of antibodies. The antibodies may be polyclonal antibodies or may be monoclonal antibodies. The antibodies are made by methods known to those skilled in the art. Antibodies with very high affinity and specificity may be used for immunological tests for markers of cancer.

For the production of polyclonal antibodies, the immunogen, usually mixed with an adjuvant, is injected into a host animal, such as a mouse, guinea pig, rabbit, goat or horse. The injection is repeated at the same site or different sites at regular or irregular intervals. The host animal is bled periodically to assess antibody titer until it is determined that optimal titer has been reached. The antibodies are obtained either from antiserum taken from the host animal with bleeding or by somatic cell hybridization techniques known in the art.

Monoclonal antibodies, can be produced by a method known in the art, e.g. Kohler and Milstein (*Nature,* vol. 256, pp. 495-497, 1975). Generally, spleen cells are obtained from a host animal injected with the immunogen or a fragment thereof. The spleen cells are immortalized by fusion with an immortal cell line, preferably a myeloma cell line, of the same or different species as the injected host animal. The fused cells are cloned and the resulting hybridomas are screened for production of monoclonal antibodies that specifically bind the immunogen.

In the instant application, the term "an immunological assay" means any method known in the immunology art for the quantitation of substances. An example of an immunological assay is radioimmunoassay.

### STUDIES OF MRP8 and MRP14 AND OF THEIR RELEVANCE TO TUMOR BIOLOGY AND AS TUMOR MARKERS

In studies comparing 2D protein patterns from various types of lung tumors (i.e., Squamous cell carcinoma, Adenocarcinoma and Small cell carcinoma) a protein spot was identified in these tumor types which was found to be absent in the patient's normal lung tissue. This protein gave the sequence MLTELEKALN, which is 100% homologous with human MRP-8. Further, on the 2D protein patterns for lung tumors having a large MRP-8 spot, the presence of an additional low molecular weight pair of spots was noted consistent with the two forms of MRP-14 (MRP14 has two translation initiation sites situated 4 codons apart), as determined by comparison with published figures. Among the spot proteins overexpressed in lung tumors, the preferred spot proteins are MRP8 and MRP14.

### Relationship of MRP8 and MRP14 to Tumor Biology

MRP8 (IO kDa) and MRP 14 (14 kDa) are both calcium binding proteins which belong to the S 100 family of EF hand proteins, a family which consists of at least 17 members. Of interest, genes for this family of proteins have been localized to human chromosome Iq21, a region of the chromosome which is frequently rearranged in different tumor types. These proteins are proposed to play a role during differentiation, regulation of the cell cycle and cytoskeletal/membrane interactions. Both of these proteins are composed of two distinct EF-hands flanked by hydrophobic regions at either terminus and separated by a central hinge region. MRP8 has been demonstrated to mediate chemotactic activity on macrophages Interestingly, a peptide encoded by the hinge region (between the two EFhands) has been shown to specifically mediate this effect. As such, these proteins might play a role in diseases which cause chronic inflammation, including cancer.

Both the N-terminal and carboxy-terminal EF-hands are able to bind calcium, although the carboxy-terminal EF-hand does have a higher affinity. MRP8 and MRP14 have both been shown to be secreted from granulocytes and monocytes. It is presently unclear how these proteins are secreted as they do not possess a classical signal peptide. One possibility is that calcium binding may expose a hydrophobic domain which could allow an interaction with the membrane, thereby resulting in secretion of the molecules. It has been demonstrated that both MRP8 and MRP14 homodimerize and heterodimerize with each other, thus forming complexes of various molecular weights. It is presently unclear as to the precise function of each homodimer and heterodimer form.

An antibody against the cystic fibrosis antigen (an epitope formed by heterodimerization of MRP8 and MRP14) also will react positively against a 14 kDa antigen which has been shown to be MRP14. The antibody is available commercially. This antibody has been utilized for immunohistochemistry on sections of tumor tissue and corresponding normal tissue from the same patient. These stained tissue sections revealed minimal staining in the normal lung tissue. There was somewhat more reactivity in the tumor tissue, most probably due to the increased presence of infiltrative cells. Of note, however, there was a very large amount of immunoreactivity in the area of normal tissue immediately adjacent to the tumor, thus suggesting that infiltrative cells (i.e., granulocytes, monocytes and/or macrophages) were being recruited to the tumor. Moreover, whether the antibody would recognize a specific 14 kDa protein in the serum of lung tumor patients was explored, at levels greater than that which might be present in the serum of normal individuals. The serum of 14 lung tumor patients and 14 normal individuals was separated by 1 D electrophoresis, the proteins were transferred to PVDF membranes and probed with the commercial antibody. Integrated intensity analysis of reactivity in a band visualized at 14 kDa revealed markedly increased reactivity in the serum from tumor patients (n= 14; mean intensity of 0.46) as compared to that in the serum from normal individuals (n=14; mean intensity of 0.09).

These findings indicate a role for antibodies against MRP in screening for different types of cancer in which the MRP's are detected in tumor tissue.

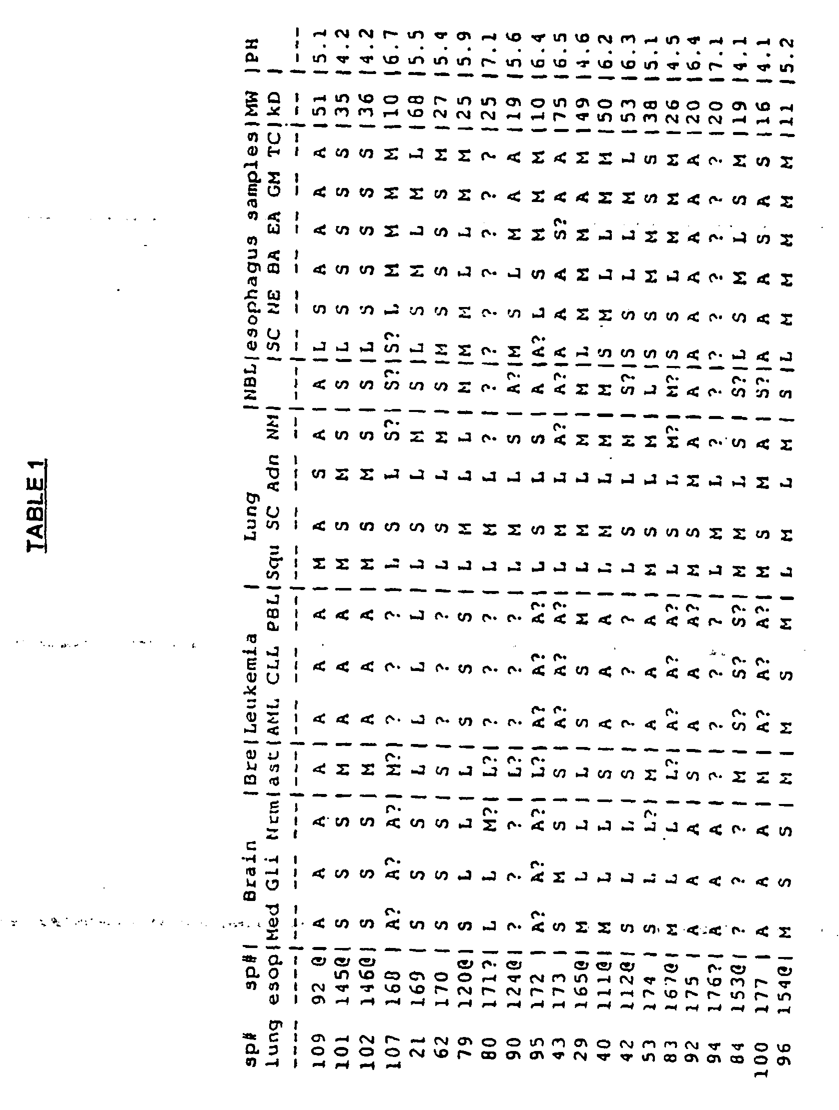

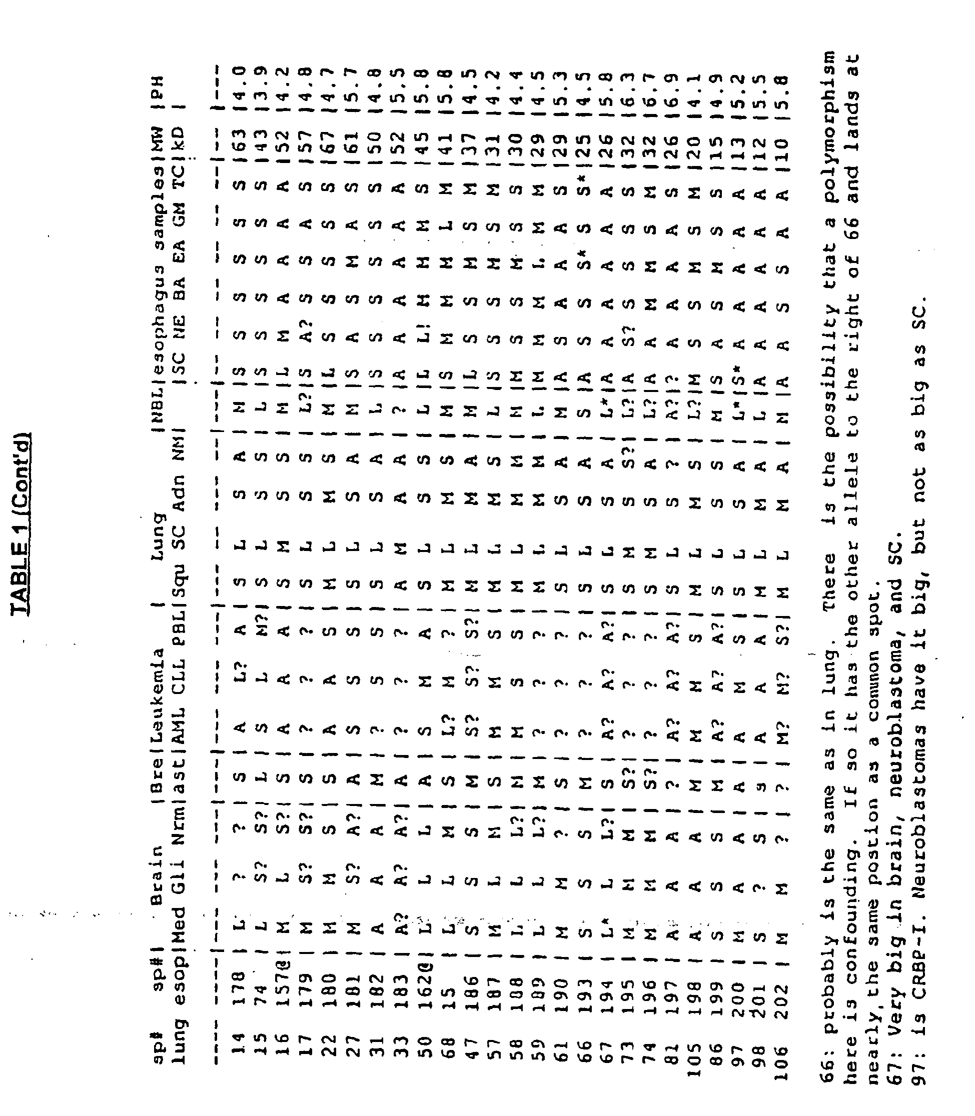

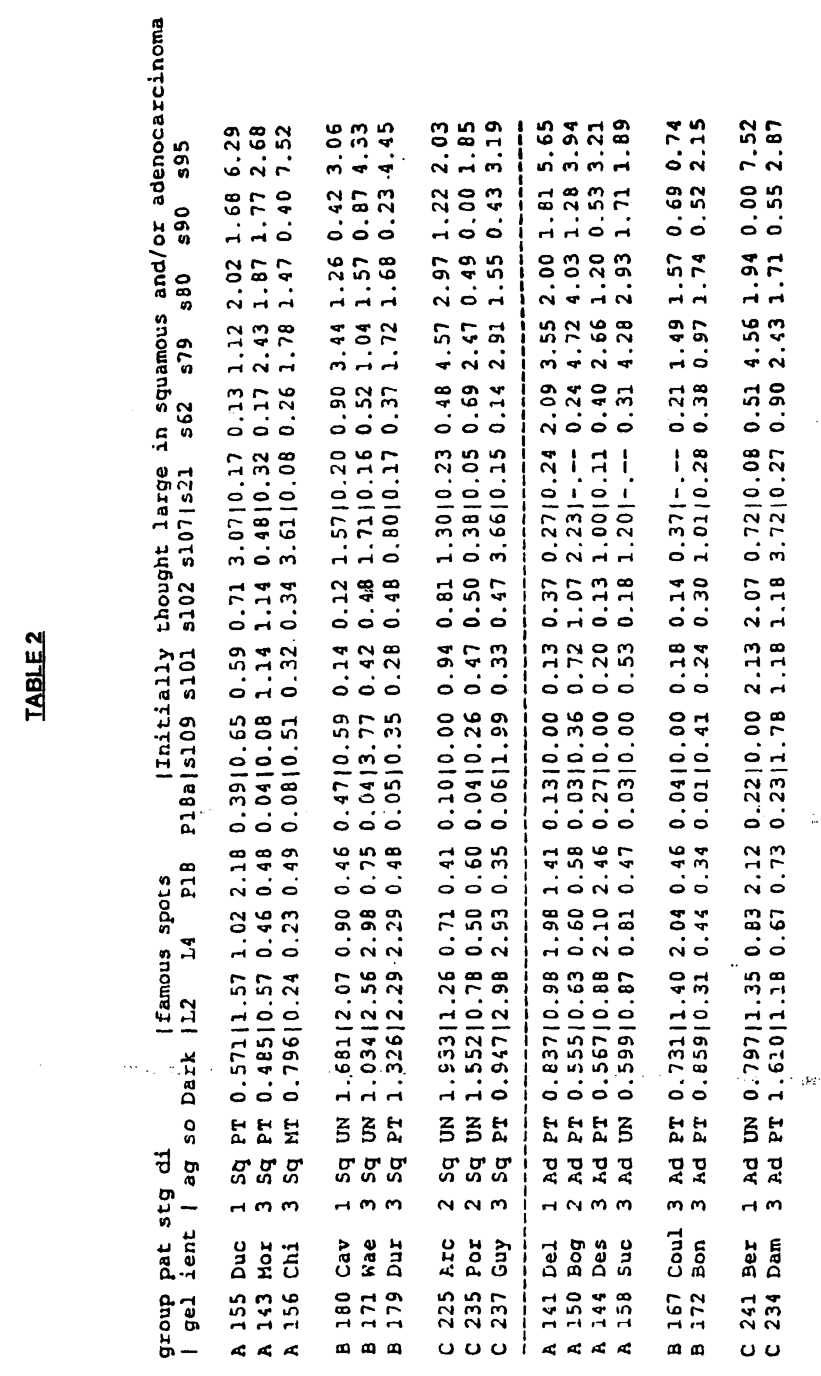

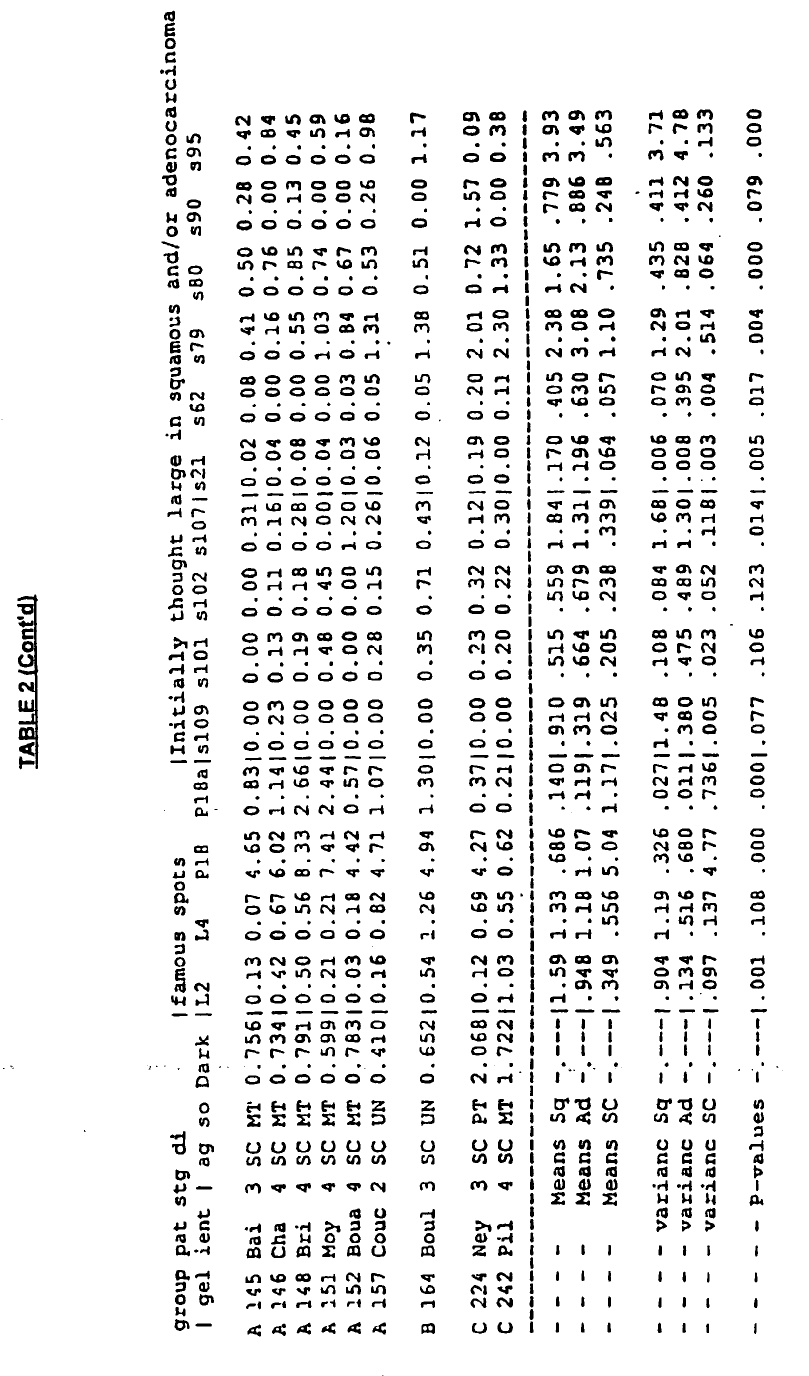

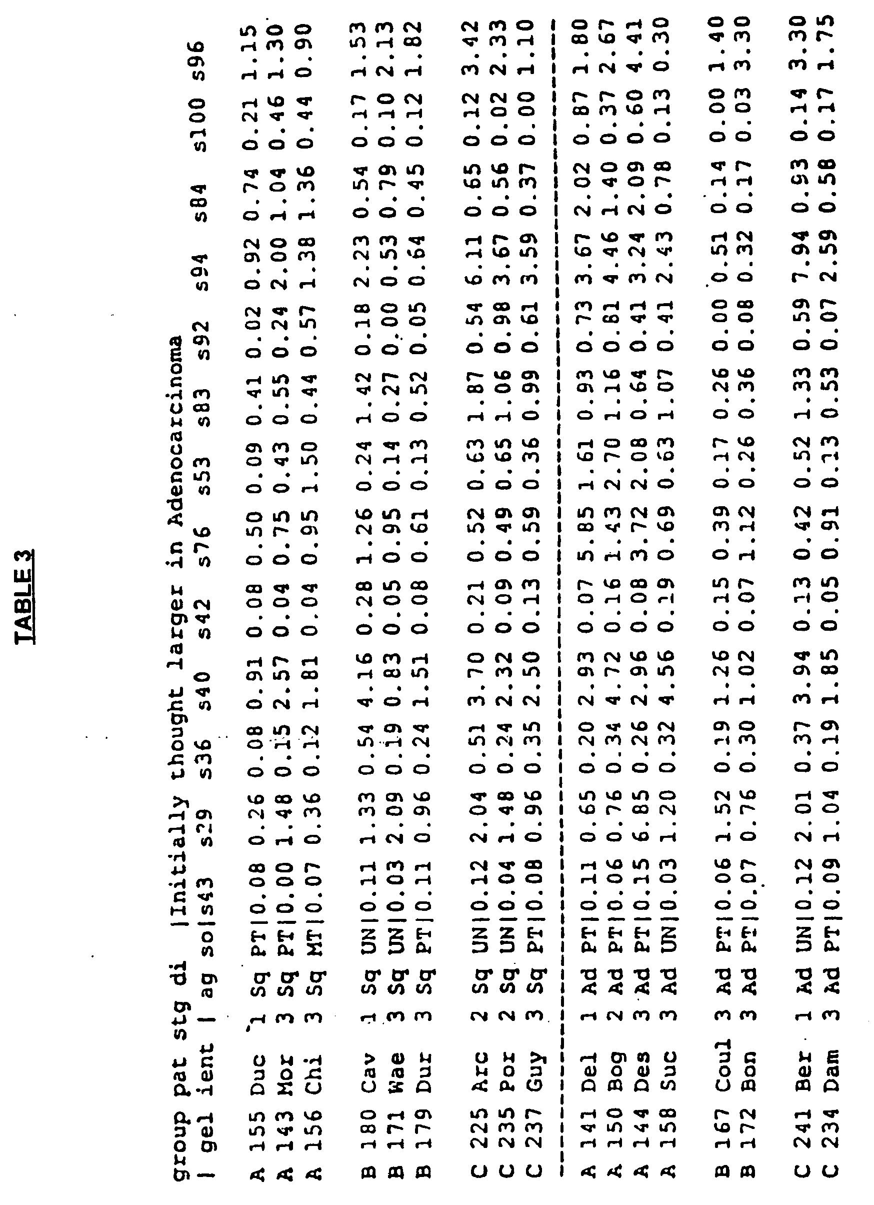

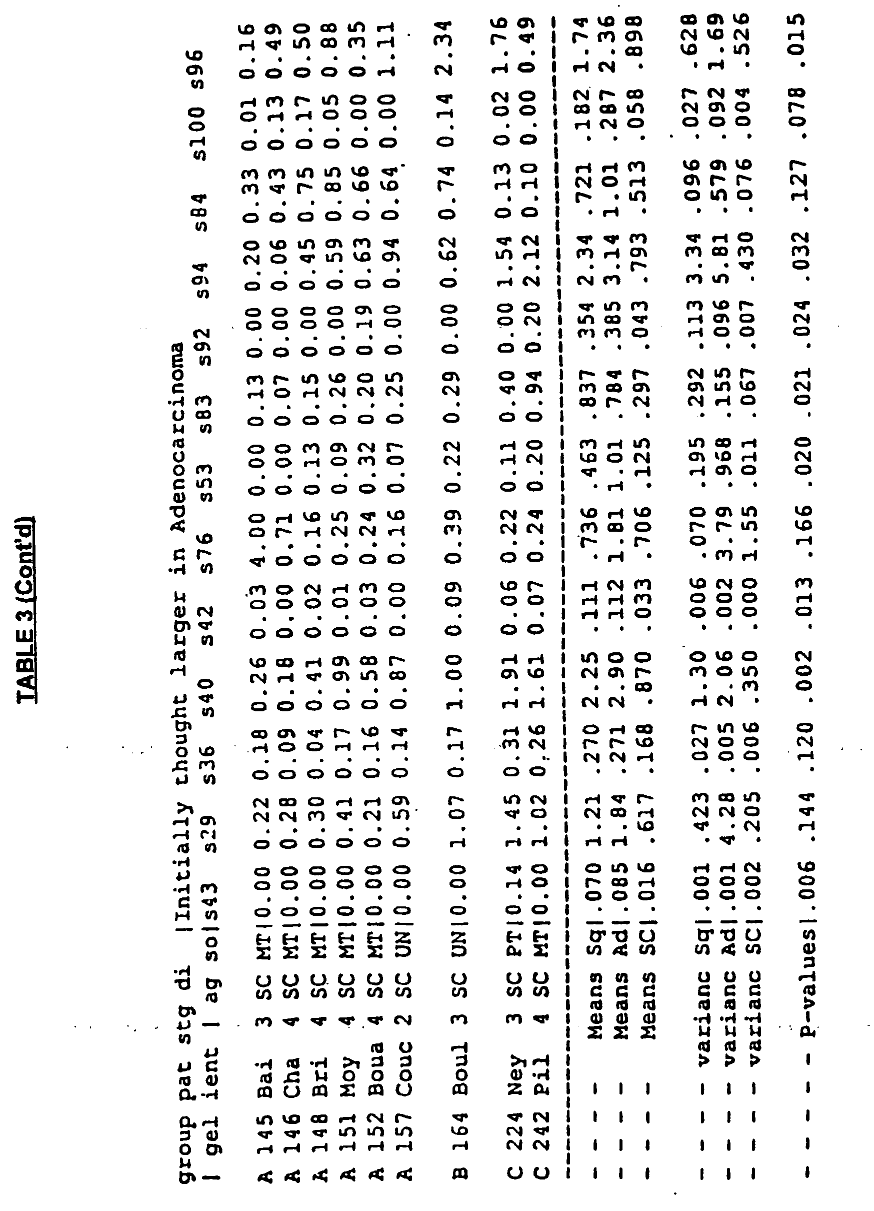

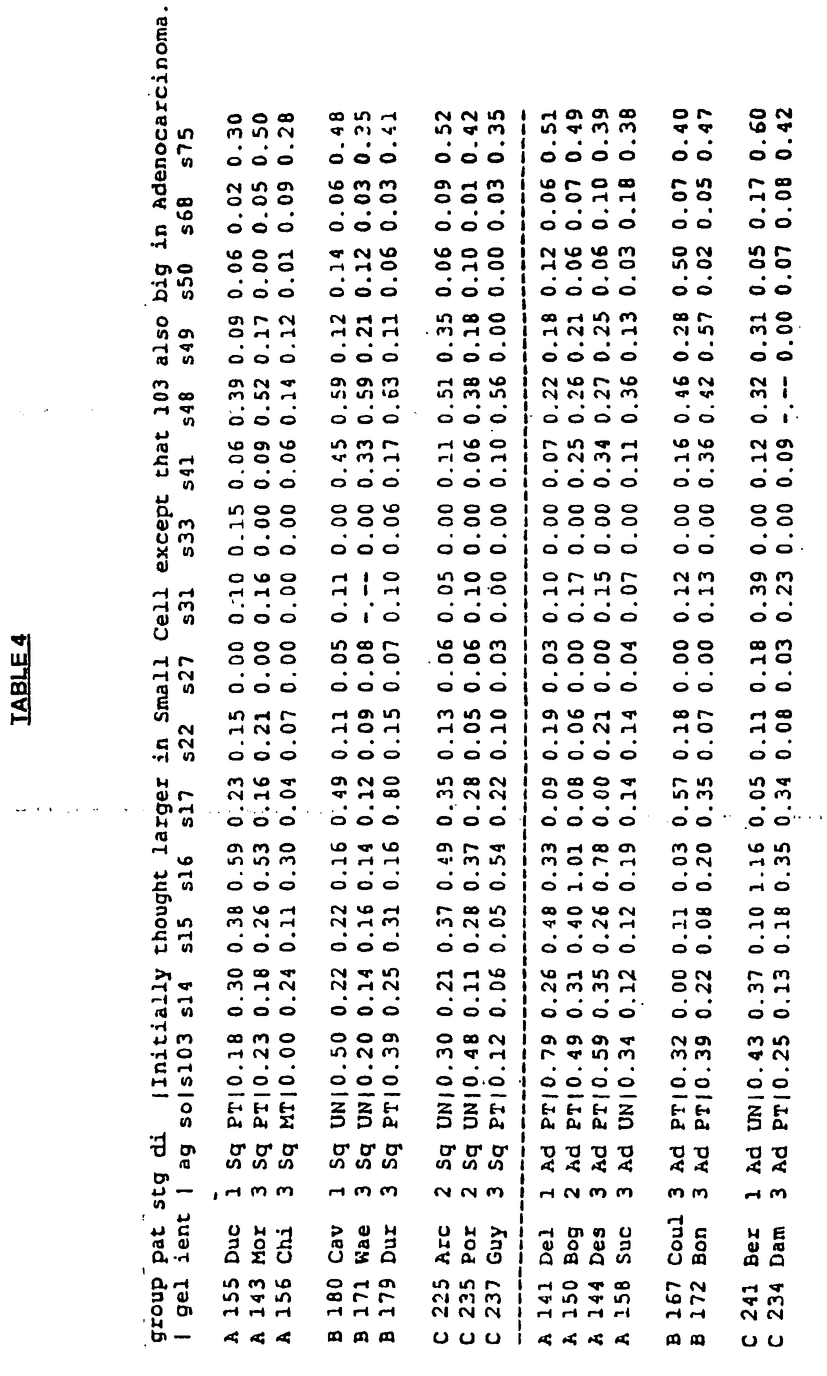

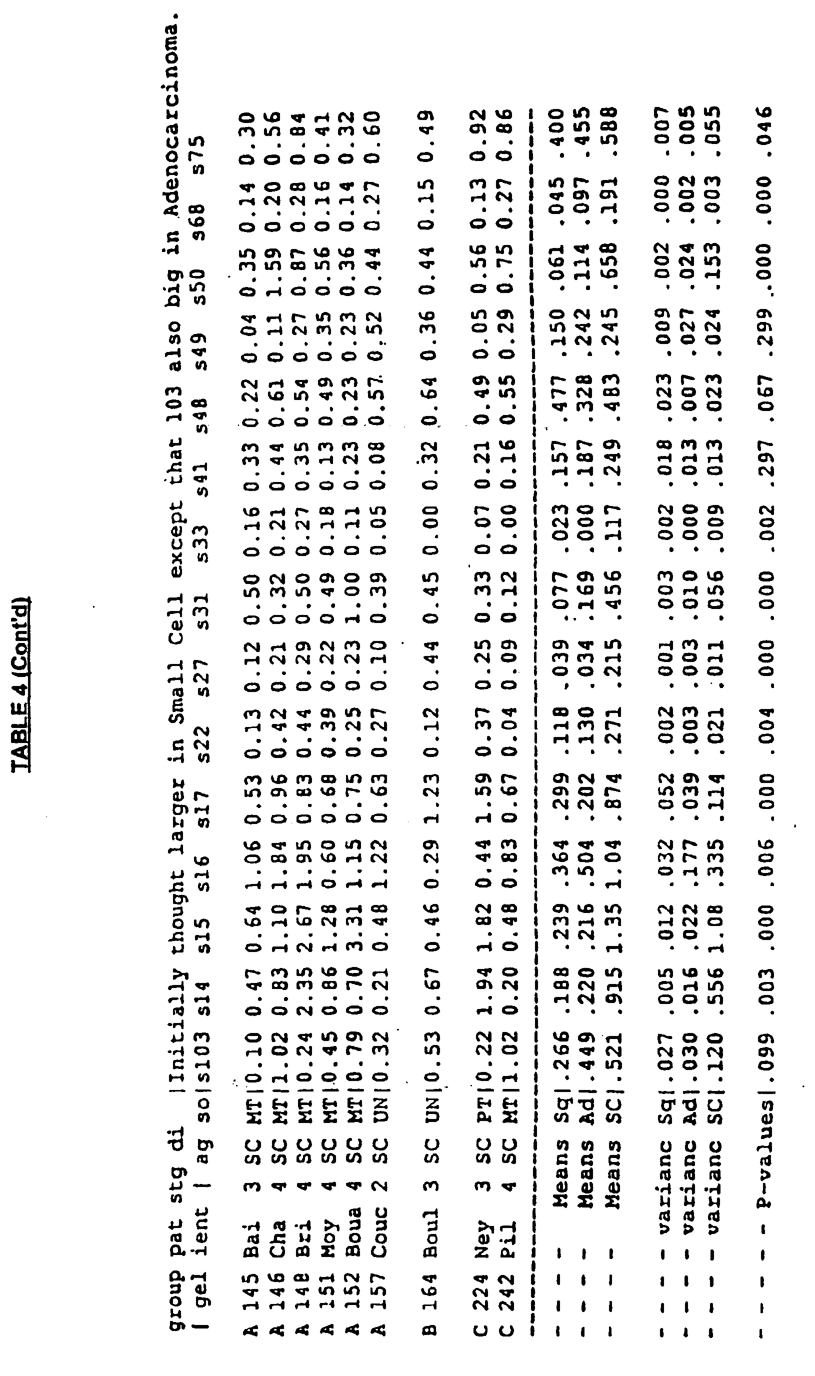

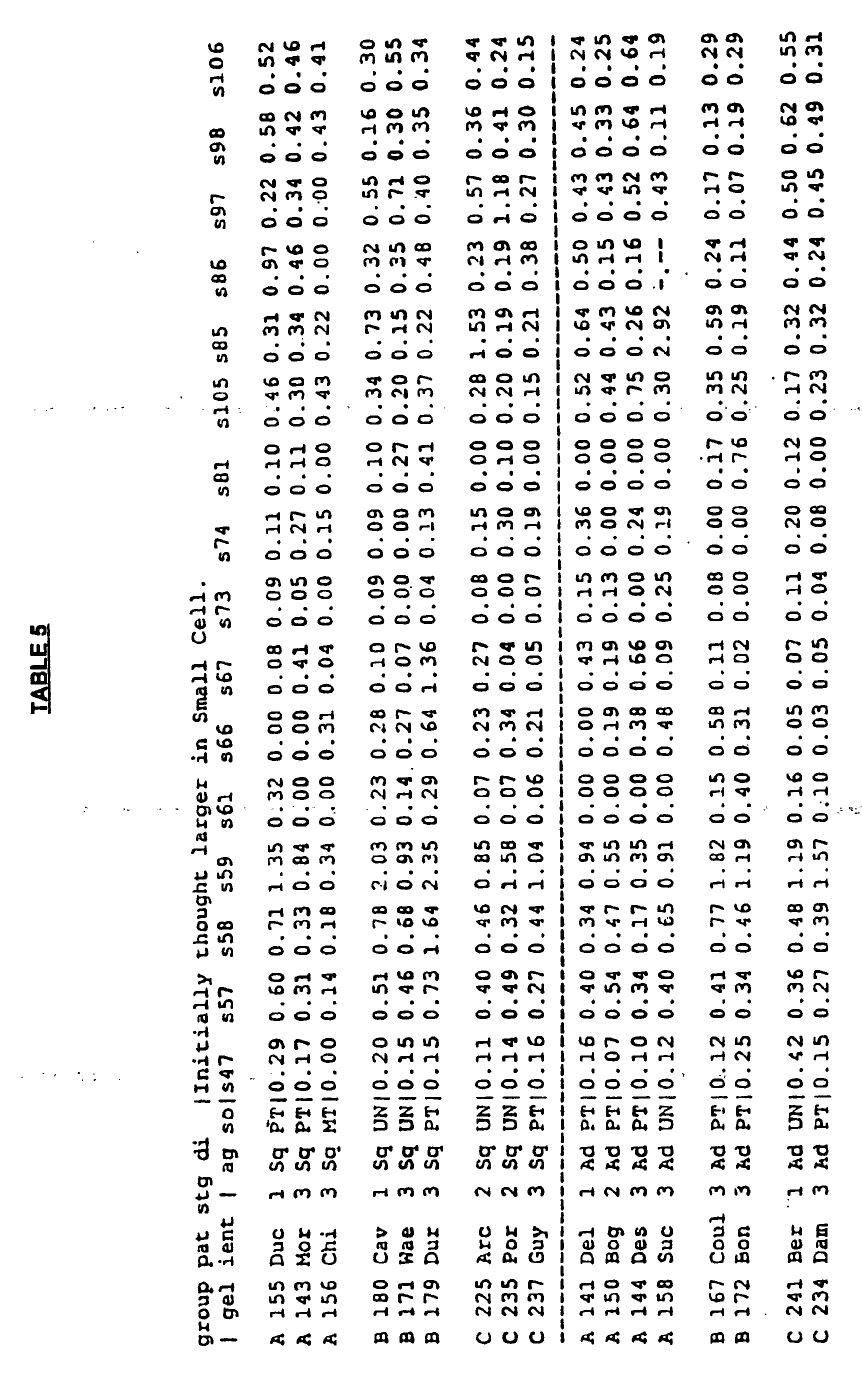

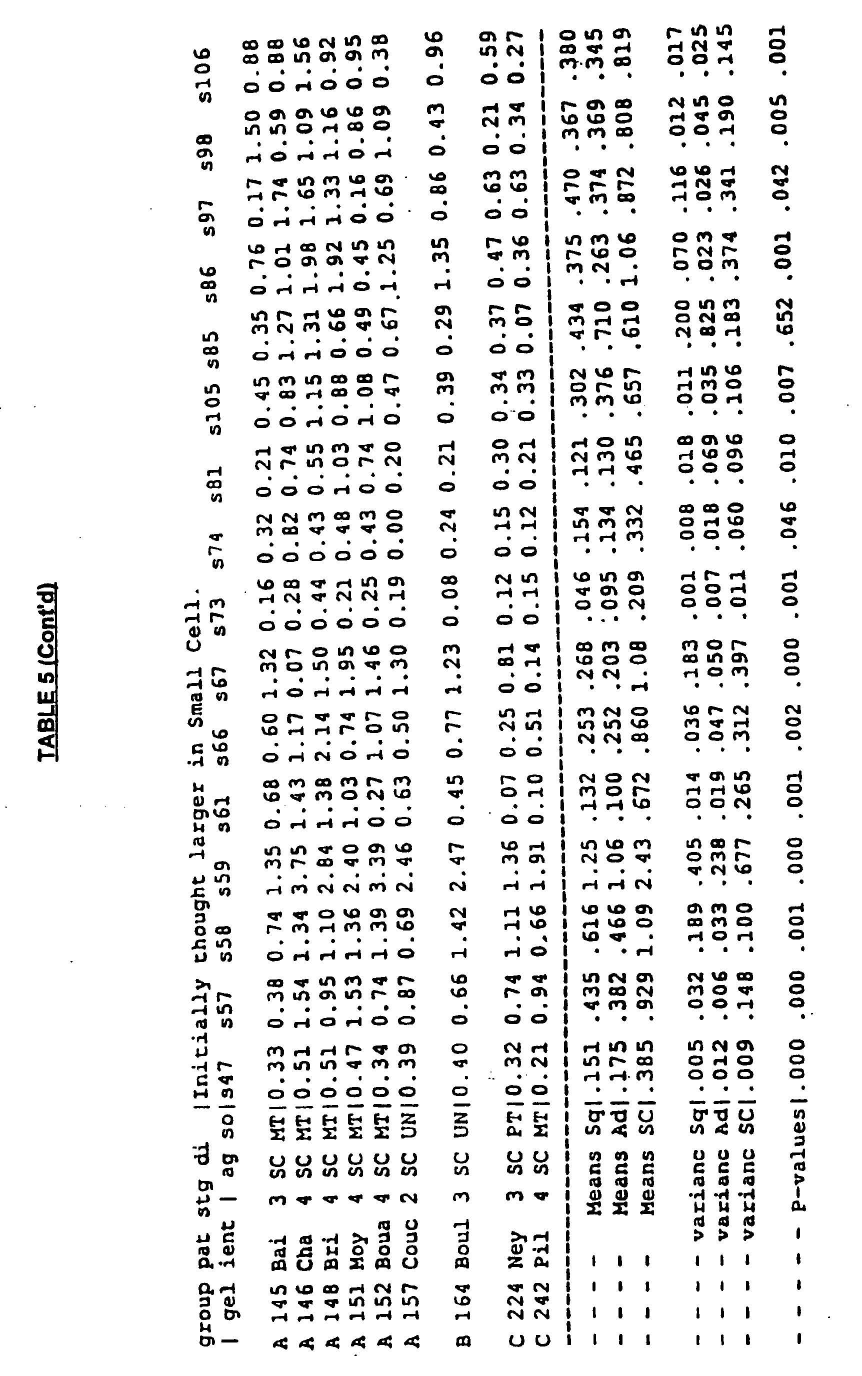

## Claims

1. A method for diagnosing lung cancer in an animal or human, comprising detecting at least one of the proteins MRP8 and MRP14 which is overexpressed in lung tumours, in a scrum sample or a tissue section from the animal or human, but excluding a diagnostic method practised on the human or animal body.

2. A method according to Claim 1, wherein an antibody specific for an epitope formed by heterodimerization of the proteins MRP8 and MRP14 is used.

3. A method according to Claim 1, comprising:
(a) treating a tissue section with an antibody specific for an epitope formed by heterodimerization of the proteins MRP8 and MRP14;
(b) washing away any unbound antibody; and
(c) determining the amount of hound antibody in the tissue section as an indication of the presence of tumour tissue.

4. A method according to Claim 1, comprising:
(a) separating proteins in a serum sample from said animal or human;
(b) transferring said proteins to a membrane;
(c) probing said proteins with an antibody specific for an epitope formed by heterodimerization of the proteins MRP8 and MRP14;
(d) determining the amount of bound antibody;
(e) integrating the intensity of reactivity in a band; and
(f) from the integrated intensity determining the presence or stage of tumour.

5. A method according to Claim 4, wherein the band is of a 14 kDa protein material.

## Patentansprüche

1. Verfahren zur Diagnostizienung von Lungenkrebs in einem Tier oder Mensch, umfassend den Nachweis von wenigstens einem der Proteine MRP8 und MRP14, welche in Lungentumoren überexprimiert sind, in einer Serumprobe oder einem Gewebeschnitt von einem Tier oder Mensch, ausgenommen Diagnostizierverfahren, die an menschlichen oder tierischen Körpern angewandt werden.

2. Verfahren nach Anspruch 1, worin, worin ein Antikörper, der für ein Epitop spezifisch ist, das durch die Heterodimerisierung der Proteine MRP8 und MRP14 gebildet wird, verwendet wird.

3. Verfahren nach Anspruch 1, umfassend:
(a) Behandlung eines Gewebeschmitts mit einem Antikörper, der für ein Epitop spezifisch ist, das durch die Heterodimerisierung der Proteine MRP8 und MRP14 gebildet wird;
(b) Abwaschen allen ungebundenen Antikörpers; and
(c) Bestimmung der Menge an gebundenem Antikörper in dem Gewebeschnitt als einen Hinweis auf die Anwesenheit von Tumorgewebe.

4. Verfahren nach Anspruch 1, umfassend:
(a) Trennung der Proteine in einer Serumprobe von dem besagten Tier oder Menschen;
(b) Transfer der besagten Proteine auf eine Membran;
(c) Sondierung der besagten Proteine mit einem Antikörper, der für ein Epitop spezifisch ist, das durch die Heterodimerisierung der Proteine MRP8 und MRP 14 gebildet wird;
(d) Bestimmung der Menge an gebundenem Antikörper;
(e) Integration der Reaktivitätsintensität in einer Bande; und
(f) Bestimmung der Anwesenheit oder des Tumorstadiums aus der integrierten Intensität.

5. Verfahren nach Anspruch 4, worin die Bande ein 14 kDa großes Proteinmaterial ist

## Revendications

1. Procédé permettant de diagnostiquer un cancer du poumon chez un animal ou un être humain, comprenant la détection d'au moins une des protéines MRP8 et MRP14, qui sont surexprimées dans les tumeurs pulmonaires, dans un échantillon de sérum ou une coupe tissulaire prélevé chez l'animal ou l'être humain, mais excluant une méthode de diagnostic pratiquée sur un corps humain ou animal.

2. Procédé selon la revendication 1, dans lequel un anticorps spécifique pour un épitope formé par hétérodimérisation des protéines MRP8 et MRP14 est utilisé.

3. Procédé selon la revendication 1, comprenant les étapes consistant à :
(a) traiter une coupe tissulaire avec un anticorps spécifique pour un épitope formé par hétérodimérisation des protéines MRP8 et MRP14 ;
(b) éliminer par lavage tout anticorps non lié ; et
(c) déterminer la quantité d'anticorps lié dans la coupe tissulaire en tant qu'indicateur de la présence d'un tissu tumoral.

4. Procédé selon la revendication 1, comprenant les étapes consistant à :
(a) séparer les protéines dans un échantillon de sérum prélevé chez ledit animal ou ledit être humain ;
(b) transférer lesdites protéines sur une membrane ;
(c) faire réagir lesdites protéines avec un anticorps spécifique pour un épitope formé par hétérodimérisation des protéines MRP8 et MRP14 ;
(d) déterminer la quantité d'anticorps lié ;
(e) intégrer l'intensité de la réactivité dans une bande ; et
(f) d'après l'intensité intégrée, déterminer la présence ou le stade d'une tumeur.

5. Procédé selon la revendication 4, dans lequel la bande est celle d'une matière protéique de 14 kDa.
